# EUROPEAN PATENT APPLICATION

(11) **EP 4 174 177 A1**
(43) Date of publication of application: **03.05.2023**
(21) Application number: 21832018.2
(22) Date of filing: 25.06.2021
(51) Int. Cl.: C12N 15/113, A61K 31/7088, A61P 31/12

(54) **CORONAVIRUS-SPECIFIC DOUBLE-STRANDED OLIGONUCLEOTIDES, AND COMPOSITION COMPRISING SAME FOR PREVENTING AND TREATING CORONAVIRUS DISEASE-19**

(30) Priority: 30.06.2020 KR 20200079766
(71) Applicant: Agency For Defense Development, Daejeon 34060 (KR); Abion Inc., Seoul 08394 (KR)
(72) Inventor: GU, Se Hun, Daejeon 34060 (KR); YU, Chi Ho, Daejeon 34060 (KR); SONG, Youngjo, Daejeon 34060 (KR); SONG, Dong Hyun, Daejeon 34060 (KR); HUR, Gyeung Haeng, Daejeon 34060 (KR); JEONG, Seong Tae, Daejeon 34060 (KR); CHOI, Jun Young, Seoul 08394 (KR); KIM, Na Young, Seoul 08394 (KR); SIM, Euni, Seoul 08394 (KR); LEE, Seung Hyeop, Seoul 08394 (KR)
(74) Representative: Ladendorf, Oliver
(86) International application number: PCT/KR2021/008011
(87) International publication number: WO 2022/005115

(57) **Abstract**

The present disclosure relates to a coronavirus-specific double-stranded oligonucleotide and a use thereof. The present disclosure induces RNA interference specific to a coronavirus, particularly, to SARS-CoV, SARS-CoV-2, and a mutant thereof so as to effectively inhibit viral infection and proliferation, and is nearly unaffected by viral variants since a conserved sequence is mainly targeted. Therefore, the double-stranded oligonucleotide of the present disclosure may be effectively used in proliferation inhibition, infection prevention, and disease treatment of the coronavirus, and particularly, may be effectively used as an agent for preventing and treating COVID-19, which is the cause of the current pandemic.

## Description

### [Technical Field]

The present disclosure relates to a coronavirus-specific double-stranded oligonucleotide and a use thereof, and more particularly, to a novel double-stranded oligonucleotide that induces RNA interference specific to SARS-CoV-2 and a composition for inhibiting proliferation of a coronavirus and/or preventing and treating coronavirus disease-19 (COVID-19) that contains the coronavirus-specific double-stranded oligonucleotide.

### [Background Art]

A coronavirus is a type of RNA virus whose genetic information is composed of ribonucleic acid (RNA). Coronaviruses cause respiratory and gastrointestinal infections in humans and animals. The coronaviruses are easily transmitted mainly by mucosal transmission, droplet transmission, and the like, and usually cause mild respiratory infections in humans, but rarely cause fatal infections. Severe acute respiratory syndrome (SARS) emerged in China in April 2003, its mortality rate was recorded as 9.6%, and as a result, many people died due to this. In 2015, Middle East respiratory syndrome (MERS) emerged in the Middle East and spread worldwide, and its mortality rate was recorded as about 36%, which was high. Coronavirus disease-19 (SARS-CoV-2) which emerged in China in December 2019 is still ongoing after the declaration of a pandemic.

Severe acute respiratory syndrome-coronavirus-2 (SARS-CoV-2) first appeared in China and spread rapidly around the world, and the WHO named the disease caused by the virus as COVID-19. According to a recent report, common symptoms include fever (98.6%), fatigue (69.6%), dry cough (59.4%), lymphopenia (70.3%), prolongation of prothrombin time (58%), and enhancement of lactate dehydrogenase (39.9%) (Wang, D., et al., JAMA, 2020). It has been reported that the SARS-CoV-2 is spread primarily through person-to-person contact via respiratory droplets in coughs and sneezes or through object surfaces contaminated by people coughing or sneezing (CDC, C.f.d.c.a.p., How COVID-19 Spreads. Coronavirus Disease 2019 (COVID-19), 2020), and it has been reported that the SARS-CoV-2 is infected asymptomatically (Yu, P., et al., J Infect Dis, 2020; Hoehl, S., et al., N Engl J Med, 2020; Bendix, A., Science alert, 2020).

As cases of SARS-CoV-2 surged around the world, the WHO declared "Public Health Emergency of International Concern (PHEIC)" on January 30, 2020, and as confirmed cases of the coronavirus continued to rise around the world, on March 11, the WHO declared COVID-19 a pandemic (global pandemic) for the third time in history following Hong Kong flu (1968) and Swine flu (2009). As of May 12, 2021, there were about more than 150 million patients worldwide, about more than 3.3 million patients have died, tens of thousands of confirmed cases have been added every day, and the number of infected patients have continuously increased. In South Korea as well, about 128,918 cases were confirmed, and 1,884 deaths were reported (see COVID-19 Dashboard by the Center for Systems Science and Engineering (CSSE) at Johns Hopkins University (JHU), statistics from Korea Disease Control and Prevention Agency (KDCA)).

Currently, despite being such a global pandemic and its seriousness, it has been treated with a comprehensive treatment in combination of antiviral treatments targeting other viruses (flu, ebola virus, and the like), antibiotics therapy, symptomatic treatments, and supportive therapy (National Library of Medicine. Clinical Features of Suspected and Confirmed Patients of 2019 Novel Coronavirus Infection). Since the comprehensive treatment as described above is only a potential treatment method using a formulation whose specificity for coronaviruses has not been proven, its clinical effect is significantly different for each patient depending on characteristics, immunity, and condition of the patient, and in particular, it is less effective for patients with underlying diseases or the old and the weak with weak immunity, such as children and elderly people. Therefore, the development of a medicine that has a clinically proven therapeutic effect specific to COVID-19 is required.

Currently, research on COVID-19 medicines based on an antiviral drug approved as medicines for other virus infections has been conducted, and specifically, antiretroviral human immunodeficiency virus I (HIV-1) protease inhibitors such as indinavir, saquinavir, and lopinavir/ritonavir, RNA polymerase inhibitors such as remdesivir, triazavirin as a new drug under investigation against Lassa fever and Ebola hemorrhagic fever, interferon beta (en) as an antiviral protein, previously identified monoclonal antibodies, and the like have been researched and clinically treated as candidate materials for COVID-19, but there is currently no finally approved medicine for COVID-19 (Paules, Catharine I., Marston, Hilary D., Fauci, Anthony S. (2020.01.23)., Coronavirus Infections-More Than Just the Common Cold; "Ural scientists have proposed testing the drug against coronavirus. Ural Federal University, 23/1/2020", and the like).

Meanwhile, RNA interference (RNAi) refers to a phenomenon in which the corresponding mRNA is specifically degraded by small RNA molecules such as microRNA (miRNA) and small interfering RNA (siRNA) and gene expression is inhibited. Since RNAi, which is used as a method for inhibiting gene expression, may clearly exhibit gene inhibition effects simply at low cost, its application fields have been diversified.

Representative nucleic acids that induce such RNA interference include about 21 RNA double strands, and typically, small interfering RNA (siRNA) consisting of 19 double helix structures and two overhangs at the 3'-end. In this case, deoxythymidine is mainly used as the nucleic acid of the two overhangs. In addition, short hairpin RNA (shRNA) consisting of a hairpin structure, which is a precursor of microRNA, is also widely used as an RNA interference derivative. In addition, recently, in particular, such an RNA interference phenomenon has been actively studied for selectively silencing viral genes, which are genes introduced from the outside rather than intracellular genes, and it has been proven that viruses and virus-induced diseases may be suppressed through the RNA interference.

As a result of diligent efforts to develop an effective double-stranded oligonucleotide that inhibits proliferation of the coronavirus through specific interference with RNA, which is a genetic material of the coronavirus, exhibits preventive and treatment effects on diseases, and is hardly affected by viral variants under such a technical background based on the RNA interference effect, the present inventors have identified a double-stranded oligonucleotide that specifically interferes with specific RNA of the leader sequence, N protein, RNA-dependent RNA polymerase (RdRp), S protein, E protein, M protein, and pplab of SARS-CoV-2, and have confirmed that the double-stranded oligonucleotide exhibits a reduction in cytopathic effect (CPE) caused by the coronavirus, a reduction in virus infection, and an inhibitory effect on viral proliferation when the double-stranded oligonucleotides are used in combination, thereby completing the present invention.

The information disclosed in the Background Art section is only for the enhancement of understanding of the background of the present invention, and therefore may not include information that forms a prior art that would already be known to those skilled in the art to which the present invention pertains.

### [Disclosure]

### [Technical Problem]

An object of the present disclosure is to provide a novel double-stranded oligonucleotide that is specific to coronavirus RNA and may inhibit expression of the coronavirus RNA with significantly high efficiency, and a double-stranded oligonucleotide including the same.

Another object of the present disclosure is to provide a pharmaceutical composition for inhibiting proliferation of a coronavirus and preventing and treating coronavirus disease.

### [Technical Solution]

In one general aspect, a coronavirus-specific RNAi-inducing double-stranded oligonucleotide contains a sense strand having one sequence selected from the group consisting of SEQ ID NOs: 1 to 61 and an antisense strand having a sequence complementary to the sense strand.

In another general aspect, there is provided a composition for inhibiting proliferation of a coronavirus containing the double-stranded oligonucleotide.

In still another general aspect, there is provided a pharmaceutical composition for preventing and/or treating coronavirus disease that contains the double-stranded oligonucleotide.

### [Advantageous Effects]

As set forth above, the double-stranded oligonucleotide according to an exemplary embodiment of the present invention induces RNA interference specific to a coronavirus, particularly, to SARS-CoV, SARS-CoV-2, and a mutant thereof so as to effectively inhibit viral infection and proliferation, and is nearly unaffected by viral variants since a conserved sequence is mainly targeted. Therefore, the double-stranded oligonucleotide according to an exemplary embodiment of the present invention may be effectively used in proliferation inhibition, infection prevention, and disease treatment of the coronavirus, and particularly, may be effectively used as an agent for preventing and treating COVID-19, which is the cause of the current pandemic.

### [Description of Drawings]

The above and other objects, features and advantages of the present invention will become apparent from the following description of preferred embodiments given in conjunction with the accompanying drawings, in which:
FIG. 1 is a result of observing a cytopathic effect on the first day on 21 types of primarily designed siRNAs.
FIG. 2 is a result of observing a cytopathic effect on the second day on the 21 types of primarily designed siRNAs.
FIG. 3 is a result of observing a cytopathic effect on the third day on the 21 types of primarily designed siRNAs.
FIG. 4 is a result of a plaque assay of the 21 types of primarily designed siRNAs.
FIGS. 5A and 5B are results of a plaque assay of 40 types of secondarily designed siRNAs.
FIG. 6 illustrates a result of a cytopathic effect and a plaque assay of Nos. 1, 9, and 14 siRNAs in which a plaque is hardly observed among the primarily designed siRNAs.
FIG. 7 is a result of real-time PCR assay targeting E protein RNA of a coronavirus.
FIG. 8 is a result of real-time PCR assay targeting RdRp RNA of a coronavirus.
FIG. 9 is a result of a plaque assay of a combination of three types (Nos. 1, 9, and 14) of siRNAs with the most excellent effect.
FIG. 10 illustrates a result of a test for a virus inhibitory effect at different concentrations of siRNA of SEQ ID NO: 14 according to Example 7 of the present disclosure.
FIG. 11 is a result of an animal test for verifying a virus proliferation inhibitory effect of siRNA of SEQ ID NO: 14 according to Example 8 of the present disclosure, which shows the number of viral RNA copies from the lung of a Syrian hamster depending on whether and how much siRNA of SEQ ID NO: 14 is treated using qRT-PCR.
FIG. 12 is a graph showing a change in body temperature of a rhesus macaque as a result of an animal test for verifying a virus proliferation inhibitory effect of siRNA of SEQ ID NO: 14 according to Example 8 of the present disclosure.
FIG. 13 is a result of an animal test for verifying a virus proliferation inhibitory effect of siRNA of SEQ ID NO: 14 according to Example 8 of the present disclosure, which shows the number of viral RNA copies from the bronchi of a rhesus macaque depending on whether and how much siRNA of SEQ ID NO: 14 is treated using qRT-PCR.

### [Best Mode]

Unless defined otherwise, all technical and scientific terms used herein have the same meanings as commonly understood by those skilled in the art to which the present invention pertains. In general, the nomenclature used herein is well known in the art and is commonly used.

Currently, COVID-19 caused by the SARS-CoV-2 virus, one of the coronaviruses, is in a global pandemic, and there are tens of thousands of confirmed cases and thousands of deaths every day. Coronaviruses are easily infected by droplets or direct/indirect contact, and the virus is transmitted through some asymptomatic people. Despite its high infectivity, there is no medicine or vaccine whose effectiveness has been clearly proven. Due to the absence of medicines and vaccines, actually, efforts on prevention through self-isolation, wearing a mask, and furthermore, house arrest in some countries have been made, and the economic and social damage caused by these efforts are significantly large. Currently, only supportive treatments that rely on the patient's immune ability and potential treatments using existing drugs whose efficacy against the coronaviruses have not be proven have been conducted for the confirmed patients.

In this situation, the present inventors have tried to develop a novel double-stranded oligonucleotide for inhibiting infection and proliferation of viruses through RNA interference specific to a coronavirus whose main genetic material is RNA.

In an exemplary embodiment of the present disclosure, in order to design a siRNA targeting a coronavirus, a target RNA is selected based on the entire gene sequence of the SARS-CoV-2 and a siRNA exhibiting an RNA interference effect specifically on the target RNA is designed. In a primary design, 21 target RNAs are selected for the leader sequence, RNA-dependent RNA polymerase (RdRp), spike (S) protein, and nucleocapsid (N) protein, selection is made based on conserved sequences with SARS-CoV, and 21 siRNAs for specifically inducing interference with each target RNA are designed so as to exhibit an RNA interference effect on coronaviruses other than SARS-CoV.

In another exemplary embodiment of the present disclosure, in order to design a more specific and effective siRNA for SARS-CoV-2 and mutant viruses of SARS-CoV-2, 40 target RNAs are selected for pplab, S, N, envelope (E), and membrane (M) proteins, selection is made on the basis of conserved sequences with no variants based on the SARS-CoV-2 virus, and 40 siRNAs that induce RNA interference specifically for each target RNA are designed.

In still another exemplary embodiment of the present disclosure, an ability of the siRNA to inhibit virus infection and virus replication and proliferation is verified by performing three methods of observation of a cytopathic effect, a plaque assay, and real-time PCR based on the designed 61 siRNAs.

Accordingly, in one aspect, the present disclosure relates to a coronavirus-specific RNAi-inducing double-stranded oligonucleotide containing a sense strand having one sequence selected from the group consisting of SEQ ID NOs: 1 to 61 and an antisense strand having a sequence complementary to the sense strand.

In the present disclosure, in a case where the nucleic acid of the double-stranded oligonucleotide is RNA, uracil (U) may be substituted with thymine (T) in the nucleotide sequences of SEQ ID NOs: 1 to 122.

In an exemplary embodiment of the present disclosure, a siRNA containing a sense strand in which thymine is substituted with uracil in SEQ ID NOs: 1 to 61 and an antisense strand complementary to the sense strand is designed. Specifically, target RNA sequences of SEQ ID NOs: 1 to 21 are selected based on conserved sequences of SARS-CoV and SARS-CoV-2, and target RNA sequences of SEQ ID NOs: 22 to 61 are selected based on conserved sequences between SARS-CoV-2 and a mutant thereof.

The term "antisense strand" in the present disclosure is a single-stranded portion having a sequence determined for use in inhibiting a target among the double strands, refers to a polynucleotide that substantially binds primarily to an Argonaute protein, serves to guide an Argonaute complex to recognize a target gene, and has a nucleic acid sequence that is substantially or 100% complementary to mRNA of an interesting target gene, and may be used interchangeably with a "guide strand". For example, the antisense strand may be entirely or partially complementary to a nucleic acid sequence of siRNA, shRNA, DsiRNA, lsiRNA, ss-siRNA, piRNA, endo-siRNA, asiRNA, or the like. The term "sense strand" is a polynucleotide that forms a double-stranded structure with the antisense strand among the double strands, serves as a passenger to help the antisense strand bind to the Argonaute protein, and has a nucleic acid sequence that is substantially or 100% identical to a target nucleic acid, and may be used interchangeably with a "passenger strand". For example, the sense strand may be entirely or partially identical to a nucleic acid sequence of siRNA, shRNA, DsiRNA, lsiRNA, ss-siRNA, piRNA, endo-siRNA, asiRNA, or the like.

In the present disclosure, any double-stranded oligonucleotide may be understood to be included in the double-stranded oligonucleotide as long as it induces an RNA interference effect, the double-stranded oligonucleotide may be, for example, siRNA, shRNA, DsiRNA, lsiRNA, ss-siRNA, piRNA, endo-siRNA, or asiRNA, and may be small interfering RNA (siRNA) in which a guide strand composed of an antisense oligonucleotide and a passenger strand composed of a sense oligonucleotide complementary thereto are complementarily hybridized as double strands or short hairpin RNA (shRNA), which is a single RNA strand having a stem-loop structure in which a guide strand sequence and a passenger strand sequence are connected by a loop, but is not limited thereto because it may vary depending on the type of nucleic acid.

The siRNA is not limited to complete pairing of double-stranded RNA parts paired with each other, and may have a hairpin structure forming a stem-loop structure, which is particularly referred to as short hairpin RNA (shRNA). Meanwhile, the double chain or step portion may include a portion that is not paired due to a mismatch (the corresponding base is not complementary), a bulge (no base corresponding to one chain), and the like. The total length is 10 to 80 bases, preferably 15 to 60 bases, and more preferably 19 to 40 bases. In addition, the loop region has no particular meaning in sequence, and only needs to have about 3 to 10 bases in order to connect sense oligonucleotides and antisense oligonucleotides at appropriate intervals. A siRNA end structure may be either a blunt end structure or a cohesive end structure. The cohesive end structure may be either a 3'-end protruding structure or a 5'-end protruding structure, and the number of protruding bases is not limited. For example, the number of bases may be 1 to 8 bases, and preferably 2 to 6 bases, and most preferably, a feature of a form cleaved by a Dicer protein is a two-base overhang.

The term "complementary binding" in the present disclosure means that the sequence of the nucleic acid fragment and a part of the fragment included in the sense or antisense strand of the double-stranded oligonucleotide hybridize and bind to each other, and in general, hybridization means, but is not limited to, Watson-Crick base pairing such as hydrogen bonding between G-C and A-T(U). The "complementary binding" means that the sense and antisense strands are bound to the extent that annealing is possible, and means that sequences of about 70 to 80% or more, preferably about 80 to 90% or more, more preferably about 90 to 95% or more, and still more preferably 95 to 99% or more of the corresponding bases of each strand may be complementary to each other or may hybridize in a completely complementary manner. However, in order to exhibit the optimal off-target inhibitory effect, the nucleic acid may further have two nucleotide overhangs at the 3'-end, mismatch base pairing with RNA of the target gene due to substitution, or generation of a bulge due to insertion. The term "bulge" refers to a part where pairing is not made due to introduction of one or more nucleotides in a double-stranded nucleic acid and is widened, and the mismatched base sequence generally refers to a case where Watson-Crick base pairing is not made between base pairs.

In an exemplary embodiment of the present disclosure, a siRNA containing a sense strand in which thymine is substituted with uracil in SEQ ID NOs: 1 to 61 and an antisense strand complementary to the sense strand is designed. Specifically, target RNA sequences of SEQ ID NOs: 1 to 21 are selected based on conserved sequences of SARS-CoV and SARS-CoV-2, and target RNA sequences of SEQ ID NOs: 22 to 61 are selected based on conserved sequences between SARS-CoV-2 and a mutant thereof. For the stability of the selected 61 siRNAs targeting coronavirus RNA, two deoxythymidine (dTdT) are attached to the 3'-ends of the sense strand (SEQ ID NOs: 1 to 61) and the antisense strand (SEQ ID NOs: 62 to 122) to prepare 61 siRNAs having 2nt overhang in which dTdT bind to the 3'-ends of sense and antisense sequences, and in another exemplary embodiment, a coronavirus infection and proliferation inhibitory effect of the siRNA is confirmed through various types of assay such as a cytopathic effect assay, a plaque assay, and real-time PCR.

Therefore, in the present disclosure, the sense strand may have a sequence selected from the group consisting of SEQ ID NOs: 1 to 61, preferably a sequence selected from the group consisting of SEQ ID NOs: 1, 5, 9, 10, 14, 16, 26, 29, 36, and 61, and more preferably a sequence of SEQ ID NO: 1, 9, 14, 26, 29, 36, or 61.

In the present disclosure, the coronavirus is preferably SARS-CoV or SARS-CoV-2, but it will be apparent that a coronavirus in which the same target RNA sequences are conserved may induce an RNA interference effect using the double-stranded oligonucleotide according to the present disclosure without limitation, thereby exhibiting a virus infection inhibitory or proliferation inhibitory effect.

In an exemplary embodiment of the present invention, when the coronavirus is a coronavirus other than SARS-CoV-2 (for example, SARS-CoV), the sequence of the sense strand is preferably selected from the group consisting of SEQ ID NOs: 1 to 21, and more preferably selected from the group consisting of SEQ ID NOs: 1, 2, 5, 8, 9, 10, 11, 12, 13, and 14 using a conserved sequence as a target RNA, but is not limited thereto.

In an exemplary embodiment of the present invention, when the coronavirus is a coronavirus other than SARS-CoV-2 (for example, SARS-CoV), the sequence of the antisense strand is preferably selected from the group consisting of SEQ ID NOs: 62 to 82, and more preferably selected from the group consisting of SEQ ID NOs: 62, 63, 66, 69, 70, 71, 72, 73, 74, and 75 using a conserved sequence as a target RNA, but is not limited thereto.

In an exemplary embodiment of the present invention, one to three, and most preferably, two deoxythymidine (dTdT) may bind to the 3'-end of the sense and/or antisense strand.

Despite the excellent effect and wide range of use of siRNA, in order for siRNA to be developed as a medicine, siRNA should be effectively delivered to target cells by improving the stability and cell delivery efficiency of siRNA in a body (FY Xie, Drug Discov. Today. 2006 Jan; 11(1-2): 67-73). In order to improve the stability in the body and solve the problem of innate immune stimulation of siRNA, research on siRNA has been actively attempted by modifying some nucleotides or backbones of siRNA to have resistance to a nuclease or using a carrier such as a viral vector, a liposome, or a nanoparticle. For example, the modification of the sense strand or antisense strand constituting the double-stranded oligonucleotide may be used in combination of one or more modifications selected from a substitution of an -OH group with -CH₃ (methyl), -OCH₃ (methoxy), -NH₂, -F (fluorine), -O-2-methoxyethyl -O-propyl, -0-2-methylthioethyl, -O-3-aminopropyl, or -O-3-dimethylaminopropyl at the 2' carbon position of a sugar structure in one or more nucleotides; a substitution of oxygen in a sugar structure in a nucleotide with sulfur; and a modification of a phosphate bond of a nucleotide to phosphorothioate, phosphorodithioate, boranophosphate, or methyl phosphonate, and a modification in the form of peptide nucleic acid (PNA), locked nucleic acid (LNA), or unlocked nucleic acid (UNA) may also be used (Ann. Rev. Med. 55, 61-65 2004; US 5,660,985; US 5,958,691; US 6,531,584; US 5,808,023; US 6,326,358; US 6,175,001; Bioorg. Med. Chem. Lett. 14:1139-1143, 2003; RNA, 9:1034-1048, 2003; Nucleic Acid Res. 31:589-595, 2003; Nucleic Acids Research, 38(17) 5761-5773, 2010; Nucleic Acids Research, 39(5) 1823-1832, 2011) .

In addition, it is known that high efficiency may be induced in vivo by linking a chemical substance or the like to an end of a siRNA passenger (sense) strand to have enhanced pharmacokinetic characteristics (J Soutschek, Nature 11; 432 (7014) : 173-8, 2004). In this case, the stability of siRNA depends on the nature of the chemical substance binding to the end of the sense (passenger) and/or antisense (guide) strand of siRNA. For example, siRNA to which a polymer compound such as polyethylene glycol (PEG) is conjugated forms a composite by interacting with an anionic phosphate group of siRNA in the presence of a cationic substance, thereby becoming a carrier having improved siRNA stability (SH Kim, J Control Release 129(2): 107-16, 2008). In particular, micelles composed of a polymer composite are extremely small in size, have a significantly uniform distribution, and are spontaneously formed, compared to another system used as a drug delivery carrier, such as microspheres or nanoparticles, and therefore, the micelles are easy to secure quality control and reproducibility of a formulation.

Therefore, in the present disclosure, a modification may be included to improve the properties of the double-stranded oligonucleotide, and preferably, one or more chemical modifications and/or PEGylation of polyethylene glycol (PEG) may be included.

In the present disclosure, the chemical modification may be one or more modifications selected from the group consisting of a substitution of an -OH group with -CH₃ (methyl), -OCH₃ (methoxy), -NH₂, -F (fluorine), -O-2-methoxyethyl -O-propyl, -O-2-methylthioethyl, -O-3-aminopropyl, or -O-3-dimethylaminopropyl at the 2' carbon position of a sugar structure in one or more nucleotides in the sense strand and/or the antisense strand; a substitution of oxygen in a sugar structure in a nucleotide with sulfur; a modification of a phosphate bond of a nucleotide to phosphorothioate, phosphorodithioate, boranophosphate, or methyl phosphonate; and a substitution with peptide nucleic acid (PNA), locked nucleic acid (LNA), or unlocked nucleic acid (UNA).

In addition, in the present disclosure, at least one selected from the group consisting of a phosphate group, a lipophilic compound, a hydrophilic compound, and a cell-permeable peptide may bind to an end of at least one of the sense strand and the antisense strand.

In particular, in the present disclosure, the hydrophilic compound may be polyethylene glycol (PEG) or hexaethylene glycol (HEG), and most preferably polyethylene glycol, but is not limited thereto.

A technology that secures stability of siRNA and improves efficient cell membrane permeability through a siRNA conjugate in which a hydrophilic substance (for example, polyethylene glycol (PEG)), which is a biocompatible polymer, is conjugated to siRNA by a simple covalent bond or a linker-mediated covalent bond so as to improve the intracellular delivery efficiency of siRNA is disclosed in Korean Patent No. 883,471, and Korean Patent No. 1,224,828 discloses a self-assembled micelle inhibitory RNA (SAMiRNA^{™}) method, which is a more advanced method.

The PEG is a synthetic polymer and is often used to increase solubility of pharmaceuticals, particularly proteins, and to control pharmacokinetics. PEG is a polydisperse material, and since a polymer in one batch is composed of the total sum of different numbers of monomers, a molecular weight thereof exhibits a Gaussian curve, and the homogeneity of the material is indicated by a polydisperse value (Mw/Mn). That is, when PEG has a low molecular weight (3 to 5 kDa), a polydisperse value of about 1.01 is exhibited, and when PEG has a high molecular weight (20 kDa), a polydisperse value of about 1.2 is exhibited, such that the higher the molecular weight, the lower the homogeneity of the material.

In an exemplary embodiment of the present invention, it is confirmed that the designed siRNA has an excellent coronavirus infection and proliferation inhibitory ability through various types of assay based on VeroE6 cells.

Accordingly, in another aspect, the present disclosure relates to a composition for inhibiting proliferation of a coronavirus containing the double-stranded oligonucleotide.

In still another aspect, the present disclosure relates to a pharmaceutical composition for preventing or treating coronavirus disease that contains the double-stranded oligonucleotide.

In an exemplary embodiment of the present invention, the composition for inhibiting proliferation of a coronavirus and the pharmaceutical composition for preventing or treating coronavirus disease may contain one or more double-stranded oligonucleotides of the present invention, and preferably one or more selected from the group consisting of double-stranded oligonucleotides containing an antisense strand having a sequence selected from the group consisting of SEQ ID NOs: 62, 70, 75, 87, 90, 97, and 122.

In an exemplary embodiment of the present invention, preferably, the coronavirus may be SARS-CoV, SARS-CoV-2, or a mutant thereof, but is not limited thereto, and any coronavirus may be used to inhibit its proliferation or prevent or treat diseases as long as it has RNA targeted by the double-stranded oligonucleotide of the present invention as genetic information.

In an exemplary embodiment of the present invention, the coronavirus disease may be, for example, severe acute respiratory syndrome (SARS), Middle East respiratory syndrome (MERS), or coronavirus disease-19 (COVID-19), but is not limited thereto.

The pharmaceutical composition may be prepared by containing at least one pharmaceutically acceptable excipient in addition to the double-stranded oligonucleotide as an active ingredient. The pharmaceutically acceptable excipient should be compatible with the active ingredient of the present invention. A saline solution, sterilized water, a Ringer's solution, a buffered saline solution, a dextrose solution, a maltodextrin solution, glycerol, ethanol, and one or a mixture of two or more of these components may be used, and other common additives such as an antioxidant, a buffer, and a bacteriostatic agent may be added as necessary. In addition, a diluent, a dispersant, a surfactant, a binder, and a lubricant may be additionally added to formulate the pharmaceutical composition into a formulation for injection such as an aqueous solution, a suspension, and an emulsion. In particular, it is preferable to provide the pharmaceutical composition formulated into a lyophilized formulation or an inhaled formulation. A method commonly known in the art to which the present invention pertains may be used to prepare a lyophilized formulation, and a stabilizer for lyophilization may be added. In particular, in the case of the inhaled formulation, the inhaled formulation may be prepared in various forms such as a dry powder form, a liquid form, and an aerosol form, and may be administered using dry powder inhalers (DPIs), nebulizers, metered-dose inhalers (MDIs), spacers, and the like.

A method of administration of the pharmaceutical composition may be determined by those skilled in the art based on symptoms and severity of disease of a patient infected with the common coronavirus. In addition, the pharmaceutical composition may be formulated into various types such as a powder, a tablet, a capsule, a solution, an injection, an ointment, and a syrup, and may be provided as a unit-dose or multi-dose container, for example, a sealed ampoule, bottle, and the like.

The pharmaceutical composition of the present disclosure may be administered orally or parenterally. Examples of an administration route of the composition according to the present disclosure include, but are not limited to, respiratory administration, intravenous administration, subcutaneous administration, intramuscular administration, intra-arterial administration, intramedullary administration, intradural administration, intracardiac administration, transdermal administration, intraperitoneal administration, intestinal administration, sublingual administration, oral administration, and topical administration. A dosage of the composition according to the present disclosure may have various ranges depending on weight, age, gender, health condition, diet, administration time and method, excretion rate, severity of disease, and the like of a patient, and may be easily determined by those skilled in the art. In addition, the composition of the present invention may be formulated into an appropriate formulation using a known technology for clinical administration.

In addition, the present disclosure provides a method for preventing or treating coronavirus disease, the method including administering the pharmaceutical composition according to various exemplary embodiments of the present invention.

Hereinafter, the present disclosure will be described in more detail with reference to Examples. These Examples are only for illustrating the present invention. It will be apparent to those skilled in the art that the scope of the present invention is not limited by these Examples.

### Example 1: SARS-Associated Coronavirus Target RNA Selection and siRNA Design

The entire gene sequence of SNU-MT039890 was obtained from the National Center for Biotechnology Information (NCBI) to design siRNAs targeting SARS-associated coronaviruses (SARS-CoV and SARS-COV-2). In order to inhibit virus replication or infection, the RNAs of the leader sequence, RNA-dependent RNA polymerase (RdRp), spike (S) protein, and nucleocapsid (N) protein were selected as targets, and siRNA candidates were obtained through siDirect (http://sidirect2.rnai.jp/) tool. Among a total of 422 siRNA candidates, a sequence conserved even in SARS-CoV was preferentially selected through siVirus (http://sivirus.rnai.jp/) so that the selected sequence could be used as a medicine for another CoV-associated infectious disease that may occur later.

However, since there was almost no conserved sequence in the case of S and N proteins, in this case, siRNAs with less off-target effects were preferentially selected. In particular, in the S protein, the design was carried out with an emphasis on RBM, which was infectious to humans, and HR2, which was a site where variants occurred the least. A total of 21 primary siRNAs including two siRNAs from the leader sequence (5'-UTR), seven siRNAs from RdRp, seven siRNAs from the S protein, and five siRNAs from the N protein were designed in the same method described above. The designed 21 primary siRNAs are as shown in Table 1.

**[Table 1]**

| **No.** | **Name** | **Target RNA** | **Target RNA sequence [Sense (5'->3')]** | **Antisense (5'->3')** | **Presence or absence of conservation** |
|---|---|---|---|---|---|
| 1 | COVID-19-LS1 | Leader sequence | CUUGUAGAUCUGUUCUCUA (SEQ ID NO: 1) | UAGAGAACAGAUCUACAAG (SEQ ID NO: 62) | ○ |
| 2 | COVID-19-LS2 | Leader sequence | CUCUAAACGAACUUUAAAA (SEQ ID NO: 2) | UUUUAAAGUUCGUUUAGAG (SEQ ID NO: 63) | ○ |
| 3 | COVID-19-N1 | N protein | CUUGAAUACACCAAAAGAU (SEQ ID NO: 3) | AUCUUUUGGUGUAUUCAAG (SEQ ID NO: 64) | X |
| 4 | COVID-19-N2 | N protein | CUGGUAAAGGCCAACAACA (SEQ ID NO: 4) | UGUUGUUGGCCUUUACCAG (SEQ ID NO: 65) | X |
| 5 | COVID-19-N3 | N protein | CAAACUGUCACUAAGAAAU (SEQ ID NO: 5) | AUUUCUUAGUGACAGUUUG (SEQ ID NO: 66) | ○ |
| 6 | COVID-19-N4 | N protein | CAAAGAUCCAAAUUUCAAA (SEQ ID NO: 6) | UUUGAAAUUUGGAUCUUUG (SEQ ID NO: 67) | X |
| 7 | COVID-19-N5 | N protein | CUAAAAAGGACAAAAAGAA (SEQ ID NO: 7) | UUCUUUUUGUCCUUUUUAG (SEQ ID NO: 68) | X |
| 8 | COVID-19-RdRp1 | RdRp | CAUAAUCAGGAUGUAAACU (SEQ ID NO: 8) | AGUUUACAUCCUGAUUAUG (SEQ ID NO: 69) | ○ |
| 9 | COVID-19-RdRp2 | RdRp | CAAUGUUGCUUUUCAAACU (SEQ ID NO: 9) | AGUUUGAAAAGCAACAUUG (SEQ ID NO: 70) | ○ |
| 10 | COVID-19-RdRp3 | RdRp | CUUUAAGGAAGGAAGUUCU (SEQ ID NO: 10) | AGAACUUCCUUCCUUAAAG (SEQ ID NO: 71) | ○ |
| 11 | COVID-19-RdRp4 | RdRp | GAGUUAUGAGGAUCAAGAU (SEQ ID NO: 11) | AUCUUGAUCCUCAUAACUC (SEQ ID NO: 72) | ○ |
| 12 | COVID-19-RdRp5 | RdRp | CCUACUAUAACUCAAAUGA (SEQ ID NO: 12) | UCAUUUGAGUUAUAGUAGG (SEQ ID NO: 73) | ○ |
| 13 | COVID-19-RdRp6 | RdRp | CAUUAGUGCAAAGAAUAGA (SEQ ID NO: 13) | UCUAUUCUUUGCACUAAUG (SEQ ID NO: 74) | ○ |
| 14 | COVID-19-RdRp7 | RdRp | GGAGUAUGCUGAUGUCUUU (SEQ ID NO: 14) | AAAGACAUCAGCAUACUCC (SEQ ID NO: 75) | ○ |
| 15 | COVID-19-S1 | S protein_RBM | CUAAGGUUGGUGGUAAUUA (SEQ ID NO: 15) | UAAUUACCACCAACCUUAG (SEQ ID NO: 76) | X |
| 16 | COVID-19-S2 | S protein_RBM | CCUUUUGAGAGAGAUAUUU (SEQ ID NO: 16) | AAAUAUCUCUCUCAAAAGG (SEQ ID NO: 77) | X |
| 17 | COVID-19-S3 | S protein EP | GAUUUUGGUGGUUUUAAUU (SEQ ID NO: 17) | AAUUAAAACCACCAAAAUC (SEQ ID NO: 78) | X |
| 18 | COVID-19-S4 | S protein | CUUUAAACACGCUUGUUAA (SEQ ID NO: 18) | UUAACAAGCGUGUUUAAAG (SEQ ID NO: 79) | X |
| 19 | COVID-19-S5 | S protein_HR2 | GUAAACAUUCAAAAAGAAA (SEQ ID NO: 19) | UUUCUUUUUGAAUGUUUAC (SEQ ID NO: 80) | X |
| 20 | COVID-19-S6 | S protein_HR2 | CAAGAAUUUAAAUGAAUCU (SEQ ID NO: 20) | AGAUUCAUUUAAAUUCUUG (SEQ ID NO: 81) | X |
| 21 | COVID-19-S7 | S protein HR2 | GAAAGUAUGAGCAGUAUAU (SEQ ID NO: 21) | AUAUACUGCUCAUACUUUC (SEQ ID NO: 82) | X |

| | | | | | |
|---|---|---|---|---|---|
| * In Table 1, the presence or absence of conservation means whether or not RNA sequence for SARS-CoV virus is conserved * In Examples, siRNAs are prepared by attaching two deoxythymidine (dTdT) as an overhang to the 3'-ends of the antisense strand and the sense strand of siRNA in the table | | | | | |

In order to find a more specific and effective siRNA for SARS-CoV-2 and SARS-CoV-2 mutant, secondary siRNAs were additionally designed based on SNU-MT039890. Target proteins were expanded to and selected from pplab, S, N, Envelope (E), and Membrane (M) proteins, and candidates were selected through VIRsiRNApred (http://crdd.osdd.net/servers/virsirnapred/) tool. This program predicts the inhibition efficiency of each siRNA sequence by creating an algorithm based on about 1,700 experimentally verified siRNAs for HIV, HBV, HCV, influenza, and SARS, and thus more effective siRNAs may be obtained. The secondary siRNAs were designed with siRNAs having a score of 70 or more when the inhibition efficiency score obtained through the algorithm was scored in a range of 1 to 100, and siRNAs in which the number of human gene off-target sites was 200 or less. In addition, as mutations characteristic of RNA viruses have continued to be reported, a total of 40 secondary siRNAs were designed by prioritizing siRNAs targeting a conserved sequence in which a variant did not occur among COVID-19 variants that have been reported through Kobic (as of March 27, 20). The designed 40 secondary siRNAs are as shown in Table 2.

**[Table 2]**

| **No** | **Name** | **Target RNA** | **Target RNA sequence [Sense (5'->3')]** | **siRNA Antisense (5'->3')** | **Presence or absence of conservation** |
|---|---|---|---|---|---|
| 22 | EI_101 | Pp1a (149-167) | CUUGUGGCUUAGUAGAAGU (SEQ ID NO: 22) | ACUUCUACUAAGCCACAAG (SEQ ID NO: 83) | ○ |
| 23 | EI_102 | pp1a (196-214) | CAGCCCUAUGUGUUCAUCA (SEQ ID NO: 23) | UGAUGAACACAUAGGGCUG (SEQ ID NO: 84) | ○ |
| 24 | EI_103 | Ppla (585-603) | CUACCCUCUUGAGUGCAUU (SEQ ID NO: 24) | AAUGCACUCAAGAGGGUAG (SEQ ID NO: 85) | ○ |
| 25 | EI_104 | Ppla (2294-2312) | CAUUAGAACAACCUACUAG (SEQ ID NO: 25) | CUAGUAGGUUGUUCUAAUG (SEQ ID NO: 86) | ○ |
| 26 | EI_105 | Ppla (1135-1153) | GGACCUGAGCAUAGUCUUG (SEQ ID NO: 26) | CAAGACUAUGCUCAGGUCC (SEQ ID NO: 87) | ○ |
| 27 | EI_106 | Ppla (2401-2419) | GCCCUUGCACCUAAUAUGA (SEQ ID NO: 27) | UCAUAUUAGGUGCAAGGGC (SEQ ID NO: 88) | ○ |
| 28 | EI_107 | Ppla (4628-4646) | CAUUCCACCUAGAUGGUGA (SEQ ID NO: 28) | UCACCAUCUAGGUGGAAUG (SEQ ID NO: 89) | ○ |
| 29 | EI_108 | Ppla (5652-5670) | GGAUGGUGUUGUUUGUACA (SEQ ID NO: 29) | UGUACAAACAACACCAUCC (SEQ ID NO: 90) | ○ |
| 30 | EI_109 | Pp1a (7687-7705) | CAGCUUAUGUGUCAACCUA (SEQ ID NO: 30) | UAGGUUGACACAUAAGCUG (SEQ ID NO: 91) | ○ |
| 31 | EI_1010 | Ppla (9930-9948) | AGACAUGCUUAACCCUAAU (SEQ ID NO: 31) | AUUAGGGUUAAGCAUGUCU (SEQ ID NO: 92) | ○ |
| 32 | EI_1011 | RdRp (1043-1061) | CAGAGAGCUAGGUGUUGUA (SEQ ID NO: 32) | UACAACACCUAGCUCUCUG (SEQ ID NO: 93) | ○ |
| 33 | EI_1012 | RdRp (1668-1686) | GUAGCUGGUGUCUCUAUCU (SEQ ID NO: 33) | AGAUAGAGACACCAGCUAC (SEQ ID NO: 94) | ○ |
| 34 | EI_1013 | RdRp (2059-2077) | CUGCUUAUGCUAAUAGUGU (SEQ ID NO: 34) | ACACUAUUAGCAUAAGCAG (SEQ ID NO: 95) | ○ |
| 35 | EI_1014 | RdRp (2421-2439) | GGACCUCAUGAAUUUUGCU (SEQ ID NO: 35) | AGCAAAAUUCAUGAGGUCC (SEQ ID NO: 96) | ○ |
| 36 | EI_1015 | RdRp (2518-2536) | CCGGCUGUUUUGUAGAUGA (SEQ ID NO: 36) | UCAUCUACAAAACAGCCGG (SEQ ID NO: 97) | ○ |
| 37 | EI_201 | S protein (1031-1049) | CCACCAGAUUUGCAUCUGU (SEQ ID NO: 37) | ACAGAUGCAAAUCUGGUGG (SEQ ID NO: 98) | ○ |
| 38 | EI_202 | S protein (1139-1157) | AUGGAGUGUCUCCUACUAA (SEQ ID NO: 38) | UUAGUAGGAGACACUCCAU (SEQ ID NO: 99) | ○ |
| 39 | EI_203 | S protein (2233-2251) | GAUUCAACUGAAUGCAGCA (SEQ ID NO: 39) | UGCUGCAUUCAGUUGAAUC (SEQ ID NO: 100) | ○ |
| 40 | EI_204 | S protein (2303-2321) | CUGGAAUAGCUGUUGAACA (SEQ ID NO: 40) | UGUUCAACAGCUAUUCCAG (SEQ ID NO: 101) | ○ |
| 41 | EI_205 | S protein (2308-2326) | AUAGCUGUUGAACAAGACA (SEQ ID NO: 41) | UGUCUUGUUCAACAGCUAU (SEQ ID NO: 102) | ○ |
| 42 | EI_206 | S protein (2485-2503) | GCAGAUGCUGGCUUCAUCA (SEQ ID NO: 42) | UGAUGAAGCCAGCAUCUGC (SEQ ID NO: 103) | ○ |
| 43 | EI_207 | S protein (2845-2863) | CAAGAUGUGGUCAACCAAA (SEQ ID NO: 43) | UUUGGUUGACCACAUCUUG (SEQ ID NO: 104) | ○ |
| 44 | EI_208 | S protein (3165-3183) | AGCACCUCAUGGUGUAGUC (SEQ ID NO: 44) | GACUACACCAUGAGGUGCU (SEQ ID NO: 105) | ○ |
| 45 | EI_209 | S protein (3713-3731) | CCAGUUGCUGUAGUUGUCU (SEQ ID NO: 45) | AGACAACUACAGCAACUGG (SEQ ID NO: 106) | ○ |
| 46 | EI_2010 | S protein (3716-3734) | GUUGCUGUAGUUGUCUCAA (SEQ ID NO: 46) | UUGAGACAACUACAGCAAC (SEQ ID NO: 107) | ○ |
| 47 | EI_401 | E protein (18-36) | GGAAGAGACAGGUACGUUA (SEQ ID NO: 47) | UAACGUACCUGUCUCUUCC (SEQ ID NO: 108) | ○ |
| 48 | EI_402 | E protein (85-103) | GUUACACUAGCCAUCCUUA (SEQ ID NO: 48) | UAAGGAUGGCUAGUGUAAC (SEQ ID NO: 109) | ○ |
| 49 | EI_403 | E protein (144-162) | CGUGAGUCUUGUAAAACCU (SEQ ID NO: 49) | AGGUUUUACAAGACUCACG (SEQ ID NO: 110) | ○ |
| 50 | EI_501 | M protein (51-69) | UGAACAAUGGAACCUAGUA (SEQ ID NO: 50) | UACUAGGUUCCAUUGUUCA (SEQ ID NO: 111) | ○ |
| 51 | EI_502 | M protein (251-269) | UGGCUUGUCUUGUAGGCUU (SEQ ID NO: 51) | AAGCCUACAAGACAAGCCA (SEQ ID NO: 112) | ○ |
| 52 | EI_503 | M protein (270-288) | GAUGUGGCUCAGCUACUUC (SEQ ID NO: 52) | GAAGUAGCUGAGCCACAUC (SEQ ID NO: 113) | ○ |
| 53 | EI_504 | M protein (594-612) | CUACAGGAUUGGCAACUAU (SEQ ID NO: 53) | AUAGUUGCCAAUCCUGUAG (SEQ ID NO: 114) | ○ |
| 54 | EI_901 | N protein (53-71) | GUGGACCCUCAGAUUCAAC (SEQ ID NO: 54) | GUUGAAUCUGAGGGUCCAC (SEQ ID NO: 115) | ○ |
| 55 | EI_902 | N protein (67-85) | UCAACUGGCAGUAACCAGA (SEQ ID NO: 55) | UCUGGUUACUGCCAGUUGA (SEQ ID NO: 116) | ○ |
| 56 | EI_903 | N protein (79-97) | AACCAGAAUGGAGAACGCA (SEQ ID NO: 56) | UGCGUUCUCCAUUCUGGUU (SEQ ID NO: 117) | ○ |
| 57 | EI_904 | N protein (81-99) | CCAGAAUGGAGAACGCAGU (SEQ ID NO: 57) | ACUGCGUUCUCCAUUCUGG (SEQ ID NO: 118) | ○ |
| 58 | EI_905 | N protein (265-283) | AGAGCUACCAGACGAAUUC (SEQ ID NO: 58) | GAAUUCGUCUGGUAGCUCU (SEQ ID NO: 119) | ○ |
| 59 | EI_906 | N protein (410-428) | GAGCCUUGAAUACACCAAA (SEQ ID NO: 59) | UUUGGUGUAUUCAAGGCUC (SEQ ID NO: 120) | ○ |
| 60 | EI_907 | N protein (681-698) | GAACCAGCUUGAGAGCAAA (SEQ ID NO: 60) | UUUGCUCUCAAGCUGGUUC (SEQ ID NO: 121) | ○ |
| 61 | EI_908 | N protein (931-949) | GCUUCAGCGUUCUUCGGAA (SEQ ID NO: 61) | UUCCGAAGAACGCUGAAGC (SEQ ID NO: 122) | ○ |

| | | | | | |
|---|---|---|---|---|---|
| * In Table 2, the presence or absence of conservation means whether or not RNA sequence for SARS-CoV-2 virus mutant is conserved * In Examples, siRNAs are prepared by attaching two deoxythymidine (dTdT) as an overhang to the 3'-ends of the antisense strand and the sense strand of siRNA in Table 2 | | | | | |

Two deoxythymidine (dTdT) were attached as the primary and secondary siRNA 3'-overhangs to increase the stability of siRNA and the binding efficiency with RNA, and a total length including the overhangs was set to 21-mer.

### Example 2: Verification of siRNA Efficacy through Observation of Cytopathic Effect (CPE)

The efficacy of the primarily designed siRNA was verified in VeroE6 cells. The VeroE6 cell is a cell through which the cytopathic effect due to apoptosis that appears after SARS-CoV infection may be confirmed.

One day before, VeroE6 cells were inoculated into a 24-well plate by 1 ml in 10% FBS DMEM w/o pen-strep medium to reach 60 to 80% confluent. The next day, each of 2 µl of 100 pmol siRNA duplex and 3 µl of Lipofectamine RNAiMAX was diluted in 50 µl of Opti-MEM (Thermofisher scientific, MA, USA), and then two solutions were mixed and reacted at room temperature (RT) for 5 minutes. 50 µl of each siRNA-lipid complex was dispensed into each well so that a final siRNA concentration was 100 pmol. After incubation at 37°C under a condition of 5% CO₂ for 3 hours, the cells were washed with phosphate-buffered saline (PBS). Thereafter, dilution was performed with 1×PFU/ml of SARS-CoV2 stock to make 1×PFU/ml, 200 µl of SARS-CoV2 stock was added so that the final PFU was 200, and then incubation was performed for 1 hour and 30 minutes. After inoculation (infection), the medium was replaced with 2% FBS DMEM medium, incubation was performed at 37°C under a condition of 5% CO₂ for 3 days, and the cytopathic effect was confirmed with a microscope every day.

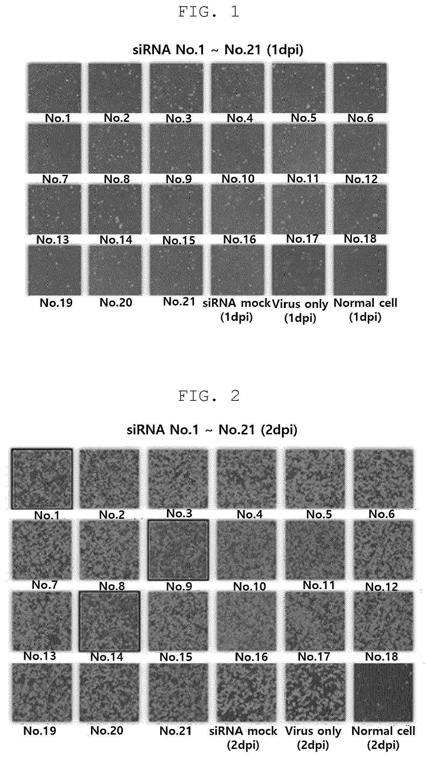

As illustrated in FIGS. 1 to 3, from the second day, the cytopathic effect could be clearly observed, and a decrease in the cytopathic effect was confirmed in all the primary siRNAs. Among the siRNAs, in the cases where No. 1 (COVID-19-LS1) siRNA, No. 9 (COVID-19-RdRp2) siRNA, and No. 14 (COVID-19-RdRp7) siRNA were treated, the cytopathic effect was the smallest, and recovery to normal cells was observed. Therefore, it was confirmed that the three primary siRNAs were the most effective.

### Example 3: Verification of Virus Infection Inhibitory Effect of siRNA through Plaque Assay

A plaque assay was performed on 21 primary siRNAs to confirm how effectively the siRNAs inhibited virus infection. On the same day that siRNA and COVID-19 virus were treated as above, overlay media obtained by mixing 1 ml of PBS with 31.5 ml of 2×MEM and 17.5 ml of Oxoid Agar was heated and then dispensed by 2 ml/well, and then incubation was performed at 37°C under a condition of 5% CO₂ for 3 days. After 3 days, 4% formaldehyde (w/methanol) was dispensed by 1.5 ml/well and then incubated for overnight (O/N) to inactivate the virus, staining with crystal violet was performed for 1 hour, and then a plaque was observed.

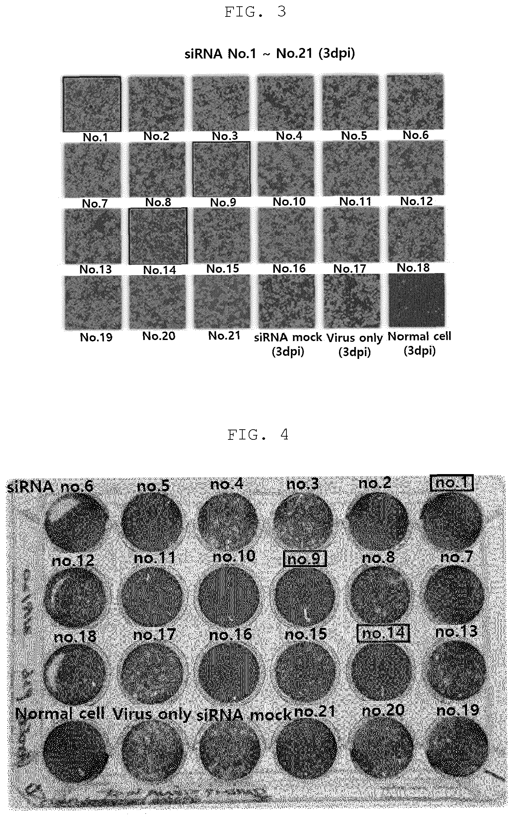

As illustrated in FIG. 4, it was confirmed that among the 21 primary siRNAs, in No. 1 (COVID-19-LS1), No. 9 (COVID-19-RdRp2), No. 10 (COVID-19-RdRp3), No. 14 (COVID-19-RdRp7), and No. 16 (COVID-19-S2), the plaque indicating infection with the virus was hardly observed, and Nos. 1, 9, and 14 siRNAs whose effects were previously verified through CPE were also effective in the plaque assay.

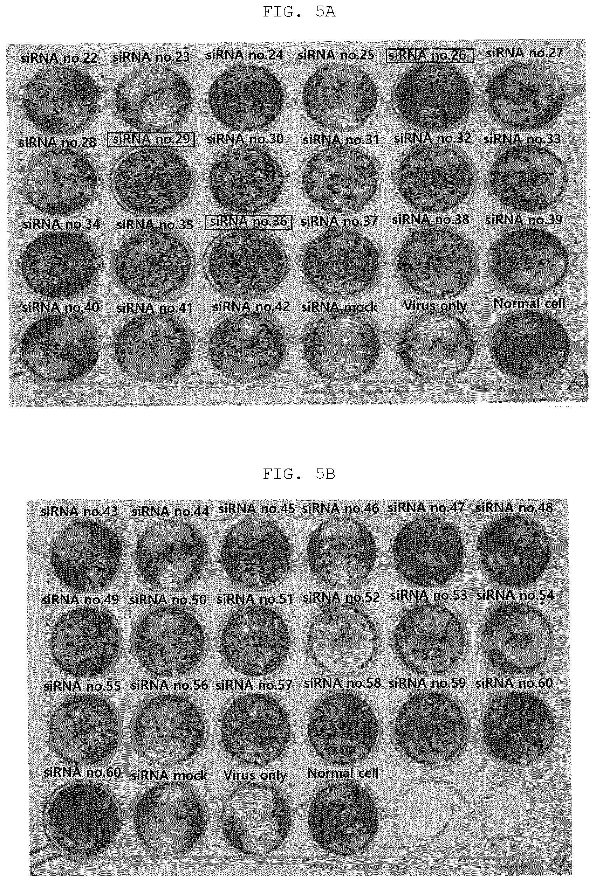

A plaque assay of the 40 secondarily designed siRNAs was performed in the same manner. As illustrated in FIGS. 5A and 5B, it was confirmed that the formation of plaque was most suppressed in the plates treated with No. 26 siRNA (EI_1O5) and No. 29 siRNA (EI_108) targeting replicase polyprotein 1a, No. 36 siRNA (EI_1015) targeting RdRp, and No. 61 siRNA (EI_908) targeting N protein.

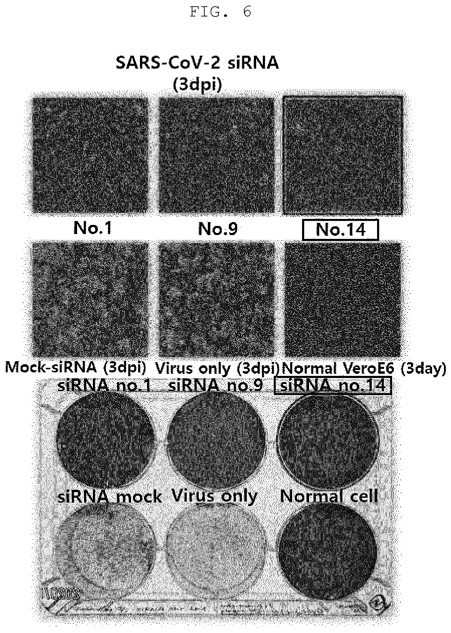

As illustrated in FIG. 6, in order to confirm once again the efficacy of Nos. 1, 9, and 14 siRNAs, which had the most excellent effect, in which the plaque indicating infection with the virus was hardly observed, among the primary siRNAs, cytopathic effect observation and a plaque assay were performed. Among them, No. 14 siRNA was confirmed to have the most excellent virus inhibitory effect.

### Example 4: Verification of Virus Replication Inhibitory Effect of siRNA through Real-Time PCR

VeroE6 cells were treated with each siRNA, on the third day, 1 ml of 2% FBS DMEM media supernatant of each cell was secured, and then RNA extraction was performed from 140 µl of the supernatant to extract SARS-CoV-2 virus RNA. In the RNA extraction, QIAamp Viral RNA mini kit was used, 560 ul of AVL buffer was added to 140 µl of the supernatant, and then voltexing was performed for 25 seconds. Incubation was performed at room temperature for 10 minutes, 560 µl of 100% EtOH was added, and voltexing was performed for 15 seconds. A liquid was added to a mini column, centrifugation was performed at 6,000xg for 1 minute, and then a solution from the collection tube was removed. 500 µl of AW1 buffer was added to each column, and centrifugation was performed at 6,000xg for 1 minute. The solution from the collection tube was discarded, 500 µl of AW2 buffer was added, and centrifugation was performed at a full speed for 3 minutes. The column was transferred to 1.5 ml of EP tube, 60 µl of AVE buffer was added, incubation was performed at room temperature for 1 minute, and then centrifugation was performed at 6,000xg for 1 minute, thereby obtaining viral RNA. cDNA was synthesized through a random primer, and then qRT PCR was performed. RT-PCR was performed by targeting E and RdRp genes using PowerChek 2019-nCoV Real-time PCR kit (Kogene, Korea), and RT-PCR was performed using QuantStudio 6 Flex (version 1.1). The PCR was performed under a condition in which a cycle of performing initial denaturation at 95°C for 10 minutes and then performing annealing and amplification at 95°C for 15 seconds and at 60°C for 1 minute was performed 40 times. A product was detected by quantifying and comparing Cr values using FAM, which was a fluorescent substance.

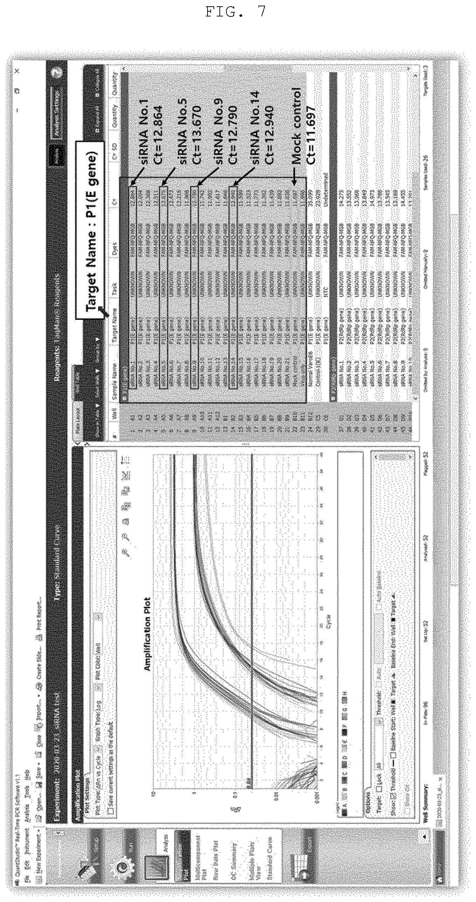

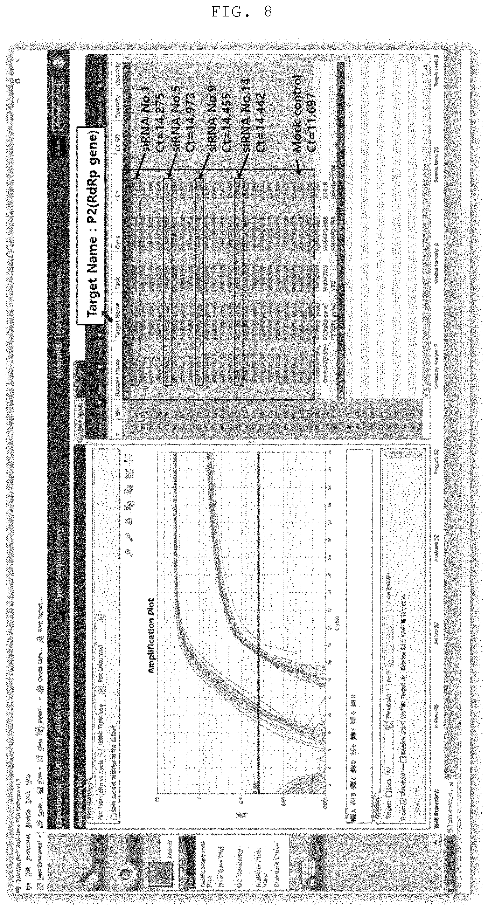

As illustrated in FIGS. 7 and 8, it was confirmed that the degree of the virus expression was relatively suppressed when the Ct values were obtained by targeting E and RdRp genes of SARS-CoV-2 and compared with a control using PowerChek2019-nCoVReal-timePCRkit manufactured by Kogene. From the fact that the Ct values of No. 1 (COVID-19-LS1) siRNA, No. 5 (COVID-19-N3) siRNA, No. 9 (COVID-19-RdRp2) siRNA, and No. 14 (COVID-19-RdRp7) siRNA were high in both E and RdRp when compared to a control group treated with siRNA mock, it was also confirmed that virus replication was inhibited when compared with another siRNA including the control.

### Example 5: Confirmation of Virus Infection Inhibitory Effect According to Combination of siRNAs

In order to the virus infection inhibitory effect according to a combination of No. 1 (COVID-19-LS1) siRNA, No. 9 (COVID-19-RdRp2) siRNA, and No. 14 (COVID-19-RdRp7) siRNA confirmed to be most effective in Examples described above, a plaque assay was performed in the same manner at 500 PFU, which was 2.5 times higher than 200 PFU, which was the virus infection concentration of Example 3. A final concentration of the siRNA combination was set to 100 nM as shown in Table 3, and the last combination was performed with a higher concentration.

**[Table 3]**

| **Mix No.** | **siRNA combination (final concentration: 100 nM)** |
|---|---|
| **1** | **LS1 (50 nM) + RdRp2 (50 nM)** |
| **2** | **LS1 (50 nM) + RdRp7 (50 nM)** |
| **3** | **RdRp2 (50 nM) + RdRp7 (50 nM)** |
| **4** | **LS1 (30 nM) + RdRp2 (30 nM) + RdRp7 (30 nM)** |
| **5** | **LS1 (50 nM) + RdRp2 (50 nM) + RdRp7 (50 nM)** |
| **mock** | **Mock control (100 nM)** |

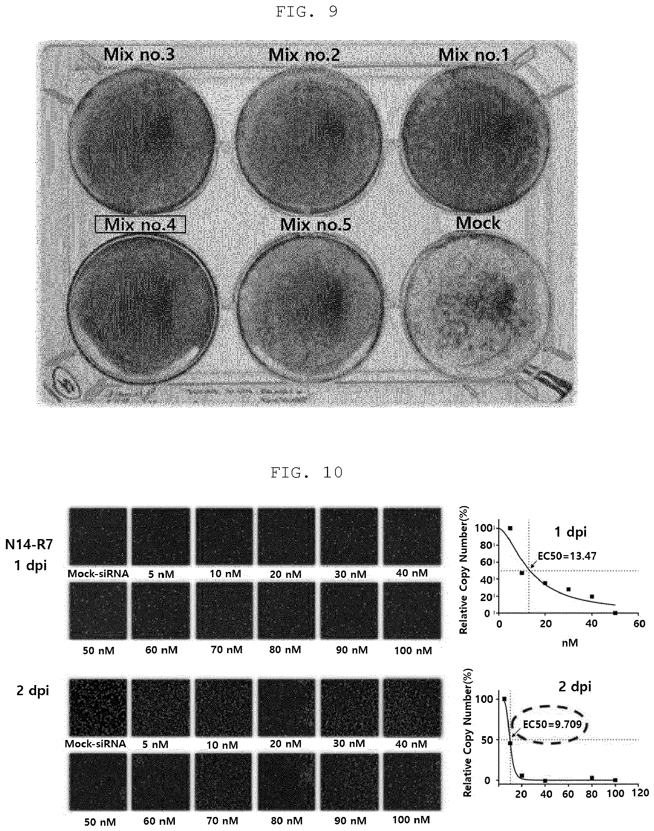

As illustrated in FIG. 9, when the siRNAs were treated in combination, it was confirmed that the occurrence of plaque was reduced in all the cases, and in particular, when all three siRNAs were treated in combination, the effect of reducing the occurrence of plaque was most excellent.

### Example 6: Selection of Final siRNAs

A total of seven siRNAs which were most effective were selected through CPE, a plaque assay, and RT-PCR, and one siRNA from the leader sequence, three siRNAs from RdRp, two siRNAs from pp1a, and one siRNA from the N protein were finally selected. The finally selected siRNAs are as shown in Table 4.

**[Table 4]**

| **No.** | **Name** | **Target RNA** | **Sense (5'->3')** | **Antisense (5'->3')** |
|---|---|---|---|---|
| 1 | COVID-19-LS1 | Leader sequence | CUUGUAGAUCUGUUCUCUA (SEQ ID NO: 1) | UAGAGAACAGAUCUACAAGDTDT (SEQ ID NO: 62) |
| 9 | COVID-19-RdRp2 | RdRp | CAAUGUUGCUUUUCAAACU (SEQ ID NO: 9) | AGUUUGAAAAGCAACAUUGDTDT (SEQ ID NO: 70) |
| 14 | COVID-19-RdRp7 | RdRp | GGAGUAUGCUGAUGUCUUU (SEQ ID NO: 14) | AAAGACAUCAGCAUACUCCDTDT (SEQ ID NO: 75) |
| 26 | EI_105 | Pp1a (1135-1153) | GGACCUGAGCAUAGUCUUG (SEQ ID NO: 26) | CAAGACUAUGCUCAGGUCCDTDT (SEQ ID NO: 87) |
| 29 | EI_108 | Pp1a (5652-5670) | GGAUGGUGUUGUUUGUACA (SEQ ID NO: 29) | UGUACAAACAACACCAUCCDTDT (SEQ ID NO: 90) |
| 36 | EI_1015 | RdRp (2518-2536) | CCGGCUGUUUUGUAGAUGA (SEQ ID NO: 36) | UCAUCUACAAAACAGCCGGDTDT (SEQ ID NO: 97) |
| 61 | EI_908 | N protein (931-949) | GCUUCAGCGUUCUUCGGAA (SEQ ID NO: 61) | UUCCGAAGAACGCUGAAGCDTDT (SEQ ID NO: 122) |

### Example 7: Inhibitory Effect at Different Concentrations of No. 14 siRNA

A test for the virus inhibitory effect at different concentrations of No. 14 siRNA which was most effective was conducted through CPE, a plaque assay, and RT-PCR. As illustrated in FIG. 10, as a result of measuring the cytopathic effect by treating the cells with No. 14 siRNA at different concentrations (5, 10, 20, 30, 40, 50, 60, 70, 80, 90, and 100 nM), a value of EC50 on the second day of cell infection was 9.709 nM, which was a more excellent numerical value than those of the effectiveness of remdesivir and chloroquine to effectively inhibit a novel coronavirus announced by the Wuhan Institute of Virology (WIV) under the Chinese Academy of Sciences. Specifically, in the cases of remdesivir and chloroquine, which were considered to have the most excellent virus inhibitory effect in the reference paper, the EC50 values were 0.77 µm and 1.13 pm, respectively. In view of this, it was confirmed that, when the inhibitory effect was determined by the EC50 value according to the present invention, the inhibitory effect was about 77.6 times superior to those of the remdesivir and chloroquine (* Reference paper: Wang M, Cao R, Zhang L, Yang X, Liu J, Xu M, Shi Z, Hu Z, Zhong W, Xiao G: Remdesivir and chloroquine effectively inhibit the recently emerged novel coronavirus (2019-nCoV) in vitro. Cell research 2020, 30(3):269-271).

### Example 8: Test for Efficacy of No. 14 siRNA on Animals

An animal test was conducted to evaluate the treatment efficacy of No. 14 siRNA, which was most effective in the intracellular experiment. Syrian hamsters and primates (rhesus macaques) were used as experimental animal models. Details of the animal experiments are shown in Table 5.

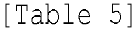

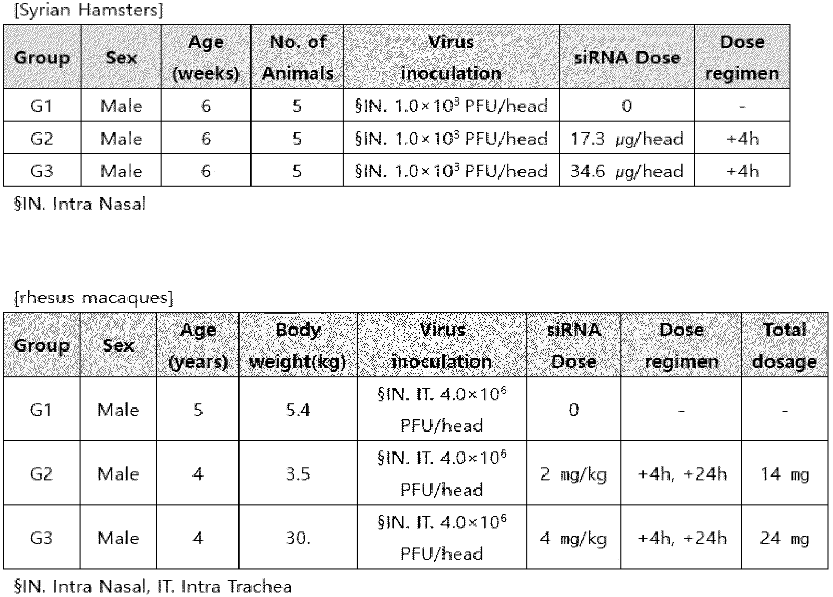

First, each group of Syrian hamsters was inoculated with 4-40,000 PFU of SARS-CoV-2 through the intranasal route, and control hamsters not treated with siRNA showed stooped posture, wrinkled fur, and mild coughing symptoms. However, in the G2 and G3 groups treated with No. 14 siRNA, no clinical symptoms of the coronavirus such as coughing were observed.

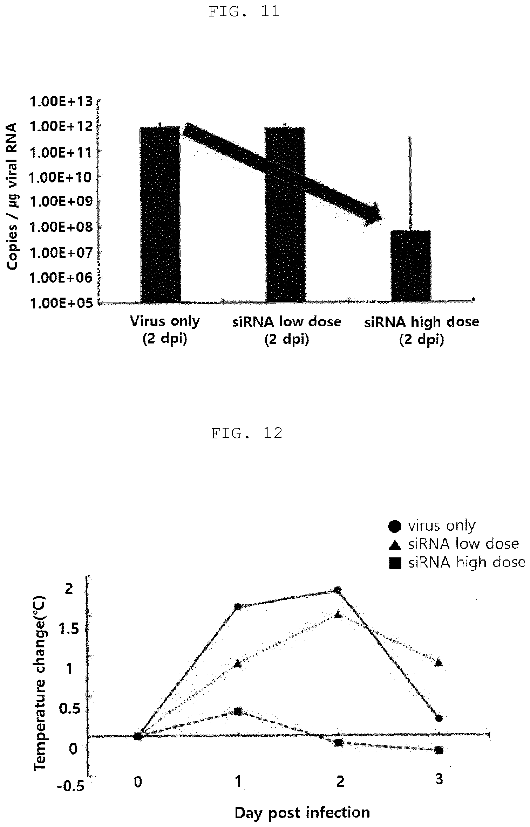

As illustrated in FIG. 11, the amount of viral RNAs in the lungs of the hamsters was assayed using qRT-PCR, and it was confirmed that the number of viral RNA copies from the lung tissues of the hamster treated with siRNA of SEQ ID NO: 14 was reduced by about 10⁴ of the virus copies compared to the control hamster.

Rhesus macaques were anesthetized and then inoculated with 4 × 10⁶ PFU of SARS-CoV-2 in a final dose of 5 ml through intratracheal (4 ml) and intranasal (1 ml) instillation. 4 hours and 24 hours after infection, the G2 group and the G3 group were treated with siRNA of SEQ ID NO: 14 in doses of 2 mg/kg and 4 mg/kg, respectively, and then observed.

As illustrated in FIG. 12, in the case of the control group, the body temperature rose to 38.6 to 40.4°C and the symptom of diarrhea appeared, but in the G3 group treated with siRNA of SEQ ID NO: 14, it was confirmed that normal body temperature was maintained for 3 days.

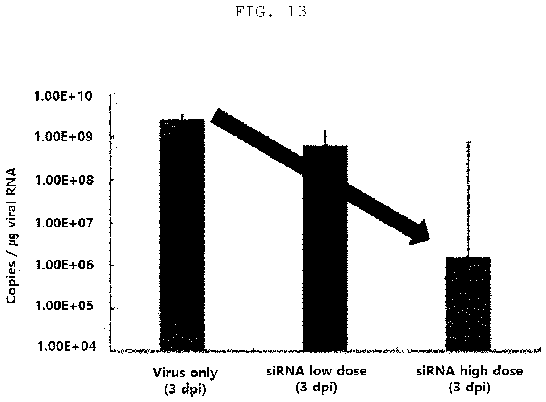

As illustrated in FIG. 13, the bronchi of the rhesus macaques were assayed using qRT-PCR, and it was confirmed that the number of viral RNA copies from the bronchial tissues of the rhesus macaque treated with siRNA of SEQ ID NO: 14 was reduced by about 10³ of the virus copies compared to the control rhesus macaque. In summary, it is demonstrated through the animal experiments that the administration of siRNA (SEQ ID NO: 14) effectively inhibits SARS-CoV-2 infection and replication in the lungs of the hamsters and the bronchi of the rhesus macaques.

## Claims

1. A coronavirus-specific RNAi-inducing double-stranded oligonucleotide comprising a sense strand having one sequence selected from the group consisting of SEQ ID NOs: 1 to 61 and an antisense strand having a sequence complementary to the sense strand.

2. The coronavirus-specific RNAi-inducing double-stranded oligonucleotide of claim 1, wherein the double-stranded oligonucleotide is selected from the group consisting of siRNA, shRNA, DsiRNA, lsiRNA, ss-siRNA, piRNA, endo-siRNA, and asiRNA.

3. The coronavirus-specific RNAi-inducing double-stranded oligonucleotide of claim 1, wherein the sense strand and the antisense strand independently consist of 19 to 31 nucleotides.

4. The coronavirus-specific RNAi-inducing double-stranded oligonucleotide of claim 1, wherein the antisense strand has a sequence selected from the group consisting of SEQ ID NOs: 62 to 122.

5. The coronavirus-specific RNAi-inducing double-stranded oligonucleotide of claim 1, wherein the antisense strand has a sequence selected from the group consisting of SEQ ID NOs: 62, 70, 75, 87, 90, 97, and 122, and
the sense strand has a sequence selected from the group consisting of SEQ ID NOs: 1, 9, 14, 26, 29, 36, and 61.

6. The coronavirus-specific RNAi-inducing double-stranded oligonucleotide of claim 1, wherein at least one of the sense strand and the antisense strand of the siRNA has one or more chemical modifications.

7. The coronavirus-specific RNAi-inducing double-stranded oligonucleotide of claim 6, wherein the chemical modification is one or more modifications selected from the group consisting of:
a substitution of an -OH group with -CH₃ (methyl), - OCH₃ (methoxy), -NH₂, -F (fluorine), -O-2-methoxyethyl -O-propyl, -O-2-methylthioethyl, -O-3-aminopropyl, or -O-3-dimethylaminopropyl at the 2' carbon position of a sugar structure in a nucleotide;
a substitution of oxygen in a sugar structure in a nucleotide with sulfur;
a modification of a phosphate bond of a nucleotide to phosphorothioate, phosphorodithioate, boranophosphate, or methyl phosphonate; and
a substitution with peptide nucleic acid (PNA), locked nucleic acid (LNA), or unlocked nucleic acid (UNA).

8. The coronavirus-specific RNAi-inducing double-stranded oligonucleotide of claim 1, wherein at least one selected from the group consisting of a phosphate group, a lipophilic compound, a hydrophilic compound, and a cell-permeable peptide binds to an end of at least one of the sense strand and the antisense strand.

9. The coronavirus-specific RNAi-inducing double-stranded oligonucleotide of claim 8, wherein the hydrophilic compound is polyethylene glycol (PEG) or hexaethylene glycol (HEG).

10. The coronavirus-specific RNAi-inducing double-stranded oligonucleotide of claim 1, wherein the coronavirus is SARS-CoV or SARS-CoV-2.

11. A composition for inhibiting proliferation of a coronavirus comprising the double-stranded oligonucleotide of any one of claims 1 to 10.

12. The composition of claim 11, wherein the composition includes one or more selected from the group consisting of double-stranded oligonucleotides containing an antisense strand having a sequence selected from the group consisting of SEQ ID NOs: 62, 70, 75, 87, 90, 97, and 122.

13. A pharmaceutical composition for preventing or treating coronavirus disease comprising the double-stranded oligonucleotide of any one of claims 1 to 10.

14. The pharmaceutical composition of claim 13, wherein the pharmaceutical composition includes one or more selected from the group consisting of double-stranded oligonucleotides containing an antisense strand having a sequence selected from the group consisting of SEQ ID NOs: 62, 70, 75, 87, 90, 97, and 122.

15. The pharmaceutical composition of claim 13, wherein the coronavirus disease is coronavirus disease-19 (COVID-19) .
